# EUROPEAN PATENT APPLICATION

(11) **EP 4 759 313 A1**
(43) Date of publication of application: **17.06.2026**
(21) Application number: 24218924.9
(22) Date of filing: 11.12.2024
(51) Int. Cl.: A61K 31/498, A61P 17/16, A61K 9/00, A61K 47/10

(54) **REGIMEN FOR OPTIMAL TREATMENT WITH A TOPICAL ALPHA-2 AGONIST**

(71) Applicant: Tarian Pharma, 06130 Grasse (FR)
(72) Inventor: Andres, Philippe, 06530 Peymeinade (FR); Czernielewski, Janusz, 06220 Vallauris (FR)
(74) Representative: Axe PI

(57) **Abstract**

The invention relates to a composition comprising an alpha2-adrenoceptor agonist, for use as a medicament, an effective dose of said composition being topically administered to a patient in need thereof in a non-continuous (stop and go) regimen. The invention is characterized in that a period of wash-out of at least two (2) days is applied between each series of daily applications during the all-treatment period to maintain the full potency of the active principle.

## Description

### FIELD OF THE INVENTION

The invention relates to the field of pharmaceutical compositions. It relates more particularly to a regimen for optimal treatment with a composition comprising an alpha2-adrenoceptor agonist, for use as a medicament, an effective dose of said composition being topically administered to a patient in need thereof in series of daily applications of said composition. The composition is preferably used in the prevention and/or treatment of skin damage resulting from a systemic treatment such as but not limited to, cancer treatment (chemotherapy, immunotherapy or targeted therapy), like local or loco-regional skin toxicity, inflammation or damage (including nerve damage or damages to the skin appendices such as hair) caused by anticancer agents.

### BACKGROUND

The adrenergic receptors (adrenoceptors) are a class of G protein (heterotrimeric guanine nucleotide-binding protein)-coupled receptors (GPCRs) that mediate the physiological actions of the endogenous catecholamines, i.e. norepinephrine and epinephrine. There are nine distinct adrenoceptors in mammalian species, which are grouped into three main classes on the basis of their amino acid sequences and biological properties: α₁ (α_{1A}, α_{1B}, and α_{1D}), α₂ (α_{2A}, α_{2B}, and α_{2C}), and β (β₁, β₂, β₃) adrenoceptors.

The α₂ adrenoceptors modulate a wide range of physiological functions, including the heart rate, blood pressure, regulation of blood glucose, insulin homeostasis, and analgesia, and they are implicated in mediating the presynaptic feedback inhibition of neurotransmitter released from noradrenergic nerve terminals.

Particularly, brimonidine is a highly selective alpha2-adrenoceptor agonist.

Topical use of brimonidine is notably approved in the treatment of glaucoma (ophthalmic solution (0.1% w/w brimonidine tartrate)) and rosacea (MIRVASO^{®} gel (0.5% w/w brimonidine tartrate)).

When topical brimonidine is applied to the skin, it reduces the persistent facial erythema of rosacea through cutaneous vasoconstriction induced by postsynaptic activation of a-adrenoceptors.

During the development and uses of these marketed products, no loss of effect overtime was reported with a high number of publications insisting that no tachyphylaxis occurred.

Tachyphylaxis is a rapid decrease in the response to a drug following its administration, leading to decrease effectiveness after repeated doses.

We also know two studies notably which assessed the efficacy and safety of topical brimonidine tartrate gel 0.5% for the treatment of erythema of rosacea. Both studies were randomized, double-blind, and vehicle-controlled, with identical design. 553 subjects with moderate to severe erythema of rosacea were randomized 1:1 to apply topical brimonidine tartrate gel 0.5% or vehicle gel once-daily for 4 weeks. No tachyphylaxis, rebound or aggravation of other disease signs were observed (Fowler et al., Efficacy and safety of once-daily topical brimonidine tartrate gel 0.5% for the treatment of moderate to severe facial erythema of rosacea: results of two randomized, double-blind, and vehicle-controlled pivotal studies. J Drugs Dermatol. 2013 Jun 1;12(6):650-6).

In a 1-year study, the long-term safety and efficacy of topical brimonidine applied daily was also assessed. Subjects with moderate to severe erythema of rosacea were instructed to apply topical brimonidine gel 0.5% once daily for 12 months. Severity of erythema and adverse events (AEs) were evaluated. Approximately 345 subject years of exposure to brimonidine gel 0.5% was achieved in the study. Effect of topical brimonidine gel 0.5% on erythema severity was observed after the first application and the durability of the effect was maintained until the end of the study at month 12, with no tachyphylaxis observed (Moore et al., Long-term safety and efficacy of once-daily topical brimonidine tartrate gel 0.5% for the treatment of moderate to severe facial erythema of rosacea: results of a 1-year open-label study. J Drugs Dermatol. 2014 Jan;13(1):56-61).

Furthermore, a multicenter, double-masked, randomized, parallel-group, active-controlled comparison study investigated the safety and efficacy of 0.2% brimonidine administered twice daily for 1 year in patients with glaucoma or ocular hypertension. Subjects instilled 0.2% brimonidine or 0.5% timolol maleate twice daily for 12 months and were examined at baseline, week 1, and months 1, 2, 3, 6, 9, and 12. No evidence of tachyphylaxis was seen in either group (Schuman et al., A 1-year study of brimonidine twice daily in glaucoma and ocular hypertension. A controlled, randomized, multicenter clinical trial. Chronic Brimonidine Study Group. Arch Ophthalmol. 1997 Jul;115(7):847-52).

In a single center, randomized double blind study evaluating the safety and efficacy of brimonidine tartrate ophthalmic solution 0.025% for the treatment of ocular redness, 60 patients were randomized. Subjects instilled treatment four times a day for 4 weeks, with clinic visits on days 15, 29, and 36 (7 days post-treatment). Brimonidine 0.025% appeared safe and effective for reduction of ocular redness, with an 8-hour duration of action, and no evidence of tachyphylaxis (McLaurin et al., Brimonidine Ophthalmic Solution 0.025% for Reduction of Ocular Redness: A Randomized Clinical Trial. Optom Vis Sci. 2018 Mar;95(3):264-271).

From these studies, in the development of these two indications (i.e. glaucoma and rosacea), the expert could conclude that no tachyphylaxis is expected during short- or long-term use of topical brimonidine.

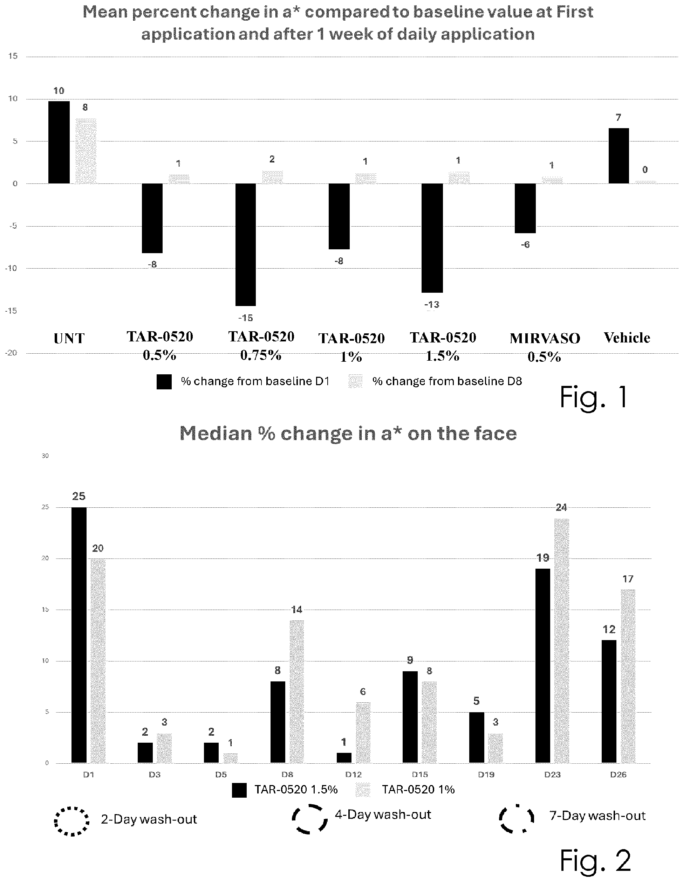

However, surprisingly, when developing a new topical formulation of brimonidine tartrate (TAR-0520 gel), a significant loss in vasoconstrictive potency was observed by the applicant. In a phase-1 study in thirty-six (36) healthy volunteers, the local tolerance of four (4) concentrations of TAR-0520 gel (0.5% to 1.5% of brimonidine tartrate) were assessed in comparison to placebo and the positive control MIRVASO^{®} (0.5% brimonidine tartrate). In addition, an assessment of the skin blanching effect (a biomarker of vasoconstriction) over a period of 12 hours after product application was performed at Day 1 and Day 8 in a subgroup of six (6) participants. The blanching effect was objectively measured by a chromameter with the parameter a* expressing the change in redness of the skin. Surprisingly, a significant loss of effect was observed at D8 compared to D1. This phenomenon affected all concentrations of TAR-0520 as well as the reference product MIRVASO^{®} (see Figure 1). It could thus be concluded that the vasoconstrictive effect induced by topical brimonidine lead to a biological response limiting the effect induced by the drug (whatever its concentration and/or formulation) on skin capillaries.

This unexpected tachyphylactic effect could negatively impact the effect of topical brimonidine in the prevention of skin toxicities induced by systemic treatments such as chemotherapies.

### PROBLEM TO BE SOLVED

The technical problem underlying the present invention is thus the provision of a new regimen for optimal treatment with a composition in a form suitable for topical administration comprising an alpha2-adrenoceptor agonist, for use as a medicament, which is costless by limiting administration of the active principle with no effect or even tachyphylaxis effect (loss of effect), should increase the treatment compliance by significantly reducing the number of administrations and keep a high clinical relevance.

The full potency of the active principle is maintained only if applications are conducted as a non-continuous (stop and go) regimen with wash-out periods.

Such a regimen is particularly of high clinical relevance if the indication is the prevention of skin toxicities induced by systemic treatments such as chemotherapies. In that case a loss of vasoconstrictive potency would lead to an increased circulation in the capillaries with higher bioavailability of chemotherapy and an increased risk of skin toxicity.

By identifying the most effective dose regimen, this also results in the best efficacy for the lowest dose applied and thus the best benefit/risk ratio for this class of drugs.

### SUMMARY OF THE INVENTION

The object of the solution according to the invention is to provide with a new non-continuous (stop and go) regimen of a composition comprising an alpha2-adrenoceptor agonist, for use as a medicament, an effective dose of said composition being topically administered to a patient in need thereof in series of daily applications of said composition, wherein a period of wash-out of at least two (2) days being applied between each series of applications during the all-treatment period.

### BRIEF DESCRIPTION OF THE DRAWINGS

The invention shall be better understood when reading the following non-restricted description, with reference to the accompanying drawings, wherein:
**Figure 1** shows the vasoconstrictive effect of four (4) increasing concentrations of brimonidine tartrate formulated in a hydrogel formulation (TAR-0520 gel) in comparison to the reference product MIRVASO^{®} gel (0.5% brimonidine tartrate), the placebo vehicle and an untreated zone (UNT), at Day 1 and at Day 8, following one (1) week of daily applications. Values are expressed as the percent change in the chromametric a* value (measuring skin redness) when comparing the mean value (among six (6) healthy volunteers) at six (6) consecutive timepoints (at 2h, 4h, 6h, 8h, 10h and 12h) to the mean baseline value at T0h. This figure shows the loss of vasoconstrictive effect (tachyphylaxis), affecting all tested active products including the reference MIRVASO^{®} (whatever the formulation and/or concentration of active principle).
**Figure 2** represents the impact of different therapeutic wash-out windows when using two different concentrations of topical brimonidine tartrate (TAR-0520 gel 1% and 1.5%) by showing the median percent change in a* value (compared to baseline value) during a 26-day study period in which twenty-six (26) healthy volunteers received TAR-0520 gel. In this study, there were nineteen (19) days of treatment in which TAR-0520 gel was applied daily on the facial area. The treatment was intermittent with no treatment on D6 and D7, no treatment on D13 and D14, no treatment from D20 to D23 (in a subgroup of thirteen (13) participants) and no treatment from D20 to D26 (in another subgroup of thirteen (13) participants). This design thus allowed the assessment of vasoconstrictive potential after two (2) days wash-out (at D8 and at D12), four (4) days of wash-out (at D23) and seven (7) days of wash-out (at D26). This figure shows that whatever the concentration, a loss of effect is confirmed as soon as Day 3, but can be reversed partially with two (2) days of wash-out, and completely with four (4) days of wash-out.

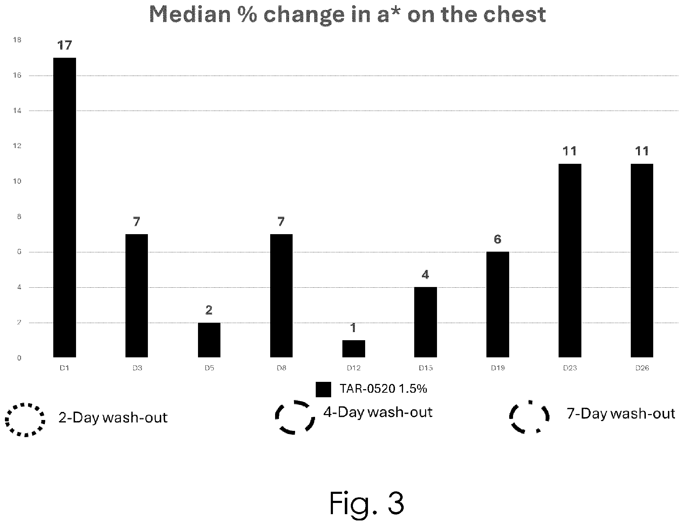

**Figure 3** represents the impact of different therapeutic wash-out windows when using topical brimonidine tartrate (TAR-0520 1.5%) by showing the percentage of change in a* value during nineteen (19) days of treatment (once daily every day except days of wash-out) on chest area, treatment having been stopped for two (2) days (at D8 and D15), for four (4) days (at D23), and for seven (7) days (at D26). This figure shows that, as for the face, a loss of effect is seen as soon as Day 3, but can be reversed partially with two (2) days of wash-out, and almost completely with four (4) days of wash-out.

### DETAILED DESCRIPTION

The object of the invention is to provide with a composition comprising an alpha2-adrenoceptor agonist, for use as a medicament, an effective dose of said composition being topically administered to a patient in need thereof, preferably a human, in series of daily applications of said composition, wherein a period of wash-out of at least two (2) days being applied between each series of applications during the all-treatment period.

Surprisingly, the inventors have shown that the full potency of the active principle is maintained only if applications are conducted as a non-continuous (stop and go) regimen with wash-out periods.

A wash-out period means a period of time set to exclude the influence of the previously used composition comprising an alpha2-adrenoceptor agonist. During the wash-out period, patients would stop applying such a composition but could still receive their systemic treatment such as but not limited to, cancer treatment (chemotherapy, immunotherapy or targeted therapy) if needed.

The composition according to the invention is preferably used in the prevention and/or treatment of skin damage induced by a systemic treatment, preferably a cancer treatment and more preferably a chemotherapy.

According to an embodiment, for example, if the chemotherapy consists of an infusion once a week, every week, the topical composition of the invention application will take place on the day of the infusion and the following two (2) to four (4) days in order to maintain a wash-out window of a few days before the next chemotherapy infusion. The number of days of topical treatment with the composition of the invention preferably depends on the speed at which the chemotherapy is cleared from the body (elimination half-life).

In the case of continuous daily chemotherapy, same stopping rules apply: the topical composition of the invention has to be stopped for a few days while the chemo agent is still administered. The principle is then that even intermittent protection still allows for a benefit.

The skin damage targeted is more particularly a local or loco-regional reaction or damage caused by anticancer agents, for example inflammation (erythema, edema), peripheral neuropathies, nail changes or hair loss induced by chemotherapy.

Chemotherapy treatment refers to the administration of chemical substances that kill, weaken or prevent the multiplication of cancer cells. These chemical substances are called chemotherapeutic agents. Illustrative examples of chemotherapeutic agents that may cause skin damage include alkylating agents, antimetabolites, topoisomerase inhibitors, microtubule assembly blockers, microtubule disassembly blockers, or protein kinase inhibitors, and more particularly busulfan, chlorambucil, cyclophosphamide, ifosfamide, melphalan, fotemustine, carmustine, temozolomide, carboplatin, cisplatin, oxiplatin, dacarbazine, methotrexate, caldribine, fludarabine, mercaptopurine, tiguanine, cytarabine, fluorouracil, capecitabine, hydroxycarbamide, doxorubicin, epirubicin, daunorubicin, idarubicin, bleomycin, dactinomycin, mitomycin C, mitoxantrone, irinotecan, topotecan, etoposide, teniposide, vinblastine, vincristine, vindesine, vinorelbine, docetaxel, paclitaxel, estramustine, alemtuzumab, amsacrine, anagrelide, bortezomib, imatinib, afatinib, erlotinib, gefitinib, dacomitinib, osimertinib, rituximab, temoporfin, and trastuzumab. The period of wash-out is preferably of more than two (2) days, more preferably at least three (3) days, between each series of applications.

According to a preferred embodiment, the period of wash-out is preferably comprised between three (3) days and seven (7) days, for example of three (3) days, four (4) days, five (5) days, six (6) days or seven (7) days, between each series of applications.

The composition used according to the invention is topically administered to a patient in need thereof, on a particular area to be treated, like for example the face, the chest, the scalp, the hand and foot, or even the nail, in series of daily applications of said composition, wherein each series of daily applications have preferably a duration of less than seven (7) days, more preferably a duration comprised between three (3) days and six (6) days.

According to a preferred embodiment, each series of daily applications have preferably a duration of three (3), four (4) or five (5) days.

According to a preferred embodiment, each series of daily applications have a similar duration, during the all-treatment period.

The topical composition used according to the invention is daily administered on one or several applications, preferably on one application.

The composition used according to the invention preferably comprises the alpha2-adrenoceptor agonist (base) at a concentration of between 0.15% and 3.00%, preferably between 0.75% and 2.50%, more preferably between 1.00% and 1.50%, by weight of the total weight of the composition, for example at a concentration of 1.00% or 1.50%, by weight of the total weight of the composition.

In a preferred embodiment, for example, if we consider concentration of 0.5%, 1.0% and 1.5% in Brimonidine Tartrate, it corresponds to 0.33%, 0.67% and 1% in Brimonidine base, respectively.

The alpha2-adrenoceptor agonist comprised in the compositions used according to the invention is preferably brimonidine or its salts.

By salts or pharmaceutically acceptable salts is meant salts of a compound of interest that are safe and effective for topical use in mammals and that possess a desired biological activity. Pharmaceutically acceptable salts include salts of acidic or basic groups present in the specified compounds. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, ptoluenesulfonate and pamoate (i.e., 1,1'-methylene-bis-(2-hydroxy-3-naphthoate)). Certain compounds used in the present invention can form pharmaceutically acceptable salts with various amino acids. Suitable base salts include, but are not limited to, aluminum, calcium, lithium, magnesium, potassium, sodium, zinc, and diethanolamine salts. For a review on pharmaceutically acceptable salts, see BERGE et al., 66 J. PHARM. SCI. 1-19 (1977).

As used herein, the term "hydrate" refers to a compound of interest, or a pharmaceutically acceptable salt thereof, which further comprises a stoichiometric or non-stoichiometric amount of water bound thereto by non-covalent intermolecular forces.

Preferably, the brimonidine salt used in the composition used according to the invention is brimonidine tartrate.

The concentration of the alpha2-adrenoceptor agonist, preferably of brimonidine or its salts, more preferably of brimonidine tartrate, and the dose thus applied can be advantageously adapted according to the area of application.

Indeed, the barrier constituted by the skin is thicker, in particular in terms of stratum corneum to be crossed at the level of the feet, in particular the soles of the feet, and the hands, in particular the palms of the hands, than the scalp, the rest of the body and in particular the chest, having an intermediate thickness. Thus, for the same dose, the concentration preferably used on the body, for example the chest, can advantageously be 0.75-1.50% w/w, higher than that used at the level of the scalp and lower than that used at the level of the feet and hands.

The composition used according to the invention can be applied to all areas of the skin where skin damage resulting from exposure to chemotherapeutic agents may appear, for example the scalp, face, neck, shoulders, back (upper and lower), torso, abdomen, genitals, arms, legs, hands and feet, or nails.

Skin damage resulting from exposure to a chemotherapeutic agent and in particular from chemotherapy treatment, more particularly refers to skin inflammation whatever its clinical expression (erythema, edema, pain).

Erythema manifests itself as a simple pinkish erythema and depending on the intensity of the exposure, it may present as a painful edematous cyanotic erythema with scaling and lasting pigmentation, or even the formation of blisters with deterioration of the general condition.

According to an advantageous embodiment, the composition used according to the invention is preferably in the form of a hydrogel, oil-in-water emulsion or water-in-oil emulsion, more preferably of a hydrogel.

According to a preferred embodiment, the composition used according to the invention preferably comprises a solvent phase comprising:
- polyethylene glycol in combination with propylene glycol and/or dimethyl sulfoxide (DMSO);
- a hydrophilic film-forming agent chosen from a Polyvinylpyrrolidone/Vinyl Acetate copolymer, polyvinylpyrrolidone in a non-crosslinked or acetate form, taken alone or in combination;
- glycerin;
and is in the form of a hydrogel.

PEGs of small molecular weights up to PEG-600, such as PEG-200, PEG-300, PEG-400, or PEG-400 SR are preferably used, more preferably PEG-400 and even more preferably PEG-400 SR advantageously promoting the stability and tolerance of the hydrogel composition according to the invention by limiting the irritation potential, eliminating polar impurities and thus reducing the excipient-active interaction (brimonidine tartrate) and the subsequent degradation of the active ingredient. Although PEG-400 or PEG-400 SR has a relatively low penetration into the stratum corneum due to its molecular weight and its high polarity (low partition coefficient), it is the PEG preferably used in the hydrogel composition used according to the invention to reduce the precipitation rate of the active ingredient on the surface of the skin and in the upper layers of the stratum corneum, for superficial solubilization. This promotes sustained delivery of brimonidine tartrate into the viable layers of the skin and specifically into the vasculature of the dermal plexus where the target site of brimonidine tartrate is located. PEG-400 or PEG-400 SR has adequate solubility to promote enhanced retention of brimonidine tartrate in solution at the skin surface and in the upper layers of the stratum corneum.

The hydrogel composition preferably used according to the invention comprises PEG at a concentration of between 1% and 20% by weight of the total weight of the composition, preferably between 5% and 15%, more preferably 10%.

Propylene glycol (PG, 1,2-propanediol) is a clear, colorless, hygroscopic liquid that is widely used as a solvent and preservative in a variety of parenteral and non-parenteral pharmaceutical formulations. The relatively rapid penetration of PG through the stratum corneum and its volatility can deplete the residual carrier of its solvent, increase the thermodynamic activity of the active in the carrier, and thus alter the driving force of diffusion. In addition, the penetration and permeation of PG can also disrupt the lipid barrier of the stratum corneum and consequently reduce the diffusional resistance. PG thus has favorable physicochemical properties in terms of skin penetration and permeation and is absorbed through the skin. Therefore, solutes that are easily dissolved by PG (i.e. high affinity for the solvent/carrier) can advantageously benefit from improved skin penetration via a solvent resistance mechanism or traction effects.

Although data for various compounds are described in the literature as indicating that the cutaneous delivery of pharmaceutically relevant compounds can be improved by PG, it is not obvious for the skilled person to predict its characteristics in terms of stability, permeation efficiency of the active (brimonidine tartrate) and tolerability when used in a complex solvent system to obtain topical compositions according to the invention that are effective, stable and pharmaceutically acceptable. A fortiori, PG is known to penetrate the skin more rapidly than most actives and that therefore the precipitation of the active on the surface of the skin will limit its duration of action. In addition, the in vivo use concentrations of PG are generally limited to about 20% w/w or less, to avoid local irritation reactions. The choice of using PG as a solvent and penetration enhancer in the hydrogel compositions preferably used according to the invention is not easily predictable due to the solubility and other vehicle-related variables.

Preferably, the hydrogel composition used according to the invention comprises PG at a concentration of between 5% and 40% by weight of the total weight of the composition, preferably between 10% and 30%, more preferably between 15% and 25%, even more preferably 20%.

According to a preferred embodiment, PEG and PG are used in a ratio of 1:1 to 1:5, preferably 1:2.

According to a particularly preferred embodiment of the hydrogel composition used according to the invention, the PEG is taken in combination with PG only, more preferably a combination of PEG-400 SR and PG.

More preferably, the hydrogel composition used according to the invention comprises polyethylene glycol at a concentration of 10% by weight of the total weight of the composition in combination with propylene glycol at a concentration of 20% by weight of the total weight of the composition.

The hydrogel composition preferably used according to the invention comprises a hydrophilic film-forming agent chosen from a Polyvinylpyrrolidone/Vinyl Acetate copolymer, polyvinylpyrrolidone in a non-crosslinked or acetate form, taken alone or in combination, preferably a Polyvinylpyrrolidone/Vinyl Acetate copolymer (KOLLIDON VA 64^{®}).

Preferably, the hydrogel composition used according to the invention comprises the Polyvinylpyrrolidone/Vinyl Acetate copolymer (KOLLIDON VA 64^{®}) at a concentration of between 0.1% and 1.5% by weight of the total weight of the composition, preferably between 0.25% and 1.4%, more preferably between 0.5% and 1.3%, more preferably still between 0.75% and 1.25%, even more preferably 1%.

The hydrogel composition preferably used according to the invention comprises glycerin.

Glycerin (glycerol) is a well-known humectant that can increase water retention in the stratum corneum and improve hydration. Glycerin is also known and used to strengthen the normal functioning of the skin barrier, promote skin elasticity and plasticity, improve skin softness and provide anti-irritant effects. Glycerin is indeed able to attract water into the stratum corneum from the epidermis and the atmosphere. Due to its relatively high polarity, glycerin does not penetrate the skin to the same extent and depth as propylene glycol but can accumulate and form a reservoir in the hydrophilic regions of the stratum corneum and increase the water content. The interaction of glycerin with the stratum corneum, its cutaneous distribution and its relatively high solubility for brimonidine tartrate (2 times higher than that of propylene glycol) offer advantages in terms of improved cutaneous delivery and prolonged cutaneous penetration.

Preferably, the hydrogel composition used according to the invention comprises glycerin at a concentration of between 1% and 20% by weight of the total weight of the composition, preferably between 2% and 15%, more preferably between 3% and 10%, even more preferably 4%.

Particularly advantageously, the combination of PG and glycerin improves the distribution of the active ingredient, preferably brimonidine tartrate in the stratum corneum.

The hydrogel composition preferably used according to the invention comprises a gelling agent chosen from xanthan gum and hydroxycellulose (HEC), taken alone or in combination.

Preferably, the hydrogel composition used according to the invention comprises xanthan gum and/or HEC at a concentration of between 0.10% and 1.50% by weight of the total weight of the composition, preferably between 0.2% and 1%, more preferably between 0.2% and 0.75%, even more preferably respectively 0.2-0.5% for xanthan gum and 0.3-0.5% for HEC preferably taken in combination.

The hydrogel composition preferably used according to the invention also comprises oleyl alcohol and vitamin E, taken alone or in combination.

The hydrogel compositions preferably used according to the invention have a pH of between 4.0 and 6.0, preferably between 4.2 and 5.5, more preferably between 4.3 and 5.0, even more preferably 4.5.

The hydrogel compositions preferably used according to the invention have a viscosity of between 50 cps and 3000 cps, preferably between 300 cps and 2800 cps, for example approximately 500 cps, 1000 cps, 1500 cps, 1600 cps, 1700 cps, 1800 cps, 2000 cps, 2500 cps or 2750 cps.

According to another embodiment of the invention, the composition preferably used according to the invention is in the form of an oil-in-water emulsion comprising liquid crystals, and comprises an alpha2-adrenoceptor agonist, preferably brimonidine or its salts, more preferably brimonidine tartrate, in a solvent phase comprising:
- polyethylene glycol in combination with propylene glycol;
- a hydrophilic film-forming agent chosen from a Polyvinylpyrrolidone/Vinyl Acetate copolymer, polyvinylpyrrolidone in a non-crosslinked or acetate form, taken alone or in combination, preferably a Polyvinylpyrrolidone/Vinyl Acetate copolymer (KOLLIDON VA 64^{®});
- glycerin;
- an emulsifier chosen from the combination of PEG-75 stearate and glyceryl monostearate and the combination of polyoxyethylene-20 sorbitan monostearate (POLYSORBATE-60) and cetostearyl alcohol;
- an oleic acid or an oleic alcohol, preferably an oleic alcohol;
and an oily phase suitable for obtaining an oil-in-water emulsion comprising liquid crystals.

The oily phase of such an oil-in-water composition comprising liquid crystals preferably used according to the invention preferably comprises cetyl alcohol and stearyl alcohol, taken alone or in combination, preferably a combination of cetyl alcohol and stearyl alcohol, more preferably cetyl alcohol, stearyl alcohol and oleic alcohol, taken in combination, at a concentration of between 1% and 15% by weight of the total weight of the composition.

The oily phase of such an oil-in-water composition comprising liquid crystals preferably used according to the invention comprises, in place of cetyl alcohol and stearyl alcohol and preferably in combination with, a triglyceride ester of saturated caprylic and capric fatty acids of coconut/palm kernel and glycerol of plant origin, a polypropylene glycol (PPG)-1 1 stearyl ether, taken alone or in combination, preferably taken in combination, more preferably at a concentration of between 5% and 10% by weight of the total weight of the composition.

Embodiments of the present invention will now be described by way of the following examples.

### EXAMPLES

### Example 1: hydrogel formulations with different concentrations of brimonidine tartrate (= TAR-0520 gel)

| **Ingredients** | **Gel Formula# 220336.0006** | **Gel Formula# 220336.0011** | **Gel Formula# 220336.0002** | **Gel Formula# 220336.0007** |
|---|---|---|---|---|
| Brimonidine Tartrate* | 0.50 | 0.75 | 1.0 | 1.5 |
| Propylene Glycol | 20 | 20 | 20 | 20 |
| Macrogol 400 Super Refined | 10 | 10 | 10 | 10 |
| Poly (1-vinylpyrrolidone -co-vinylacetate) | 1 | 1 | 1 | 1 |
| Glycerol | 4 | 4 | 4 | 4 |
| Xanthan Gum | 0.2-0.75 | 0.2-0.75 | 0.2-0.75 | 0.2-0.75 |
| Preservative system | 0.4-1 | 0.4-1 | 0.4-1 | 0.4-1 |
| Citric Acid | q.s. pH 4.5 | q.s. pH 4.5 | q.s. pH 4.5 | q.s. pH 4.5 |
| Sodium Hydroxide | q.s. pH 4.5 | q.s. pH 4.5 | q.s. pH 4.5 | q.s. pH 4.5 |
| Purified Water | q.s. 100 | q.s. 100 | q.s. 100 | q.s. 100 |

| | | | | |
|---|---|---|---|---|
| (*) 0.5, 0.75, 1.0 and 1.5% in Brimonidine Tartrate correspond to 0.33, 0.50, 0.67 and 1% in Brimonidine base, respectively. | | | | |

### Example 2: emulsion formulations

| **Composition / N° Formula** | **19.0155-0102/F4** | **19.0155-0102/F5** |
|---|---|---|
| **Concept** | **GELOT** | **GELOT** |
| **Justification of formula** | Formula 0090 + 1% BHA | Formula 0090 + 1% BHA |
| **Ingredient** | % w/w | |
| Brimonidine Tartrate | 0.15-3 | 0.15-3 |
| Purified water | qs | qs |
| Propylene Glycol | 20 | 20 |
| Vegetable Glycerine 4810 | 5 | 5 |
| PEG-400 SR | 10 | 10 |
| KOLLIDON VA 64 | 1 | 1 |
| XANTHAN GUM | 0.2 | 0.2 |
| METHYL PARABEN | 0.2 | 0.2 |
| PHENOXYETHANOL | 1 | 1 |
| GELOT 64 | 3 | 3 |
| KOLLIWAX SA | 3 | 3 |
| MIGLYOL 812N | 2.5 | 2.5 |
| ARLAMOL PS11E-LQ-(RB) | 5 | 5 |
| KOLLICREAM OA | 2.5 | 2.5 |
| KOLLIWAX CA | 4 | 4 |
| BHA | 1 | 1 |
| PROPYL PARABENE | 0.1 | 0.1 |
| DL TOCOPHEROL | | |
| NaOH 10% | 0.18 | 0.2 |
| Total | 100.00 | 100.00 |

| **Composition / N° Formula** | **19.0155-0132/F2** | **19.0155-0133/F1** |
|---|---|---|
| **Concept** | **POLAWAX** | **POLAWAX** |
| **Justification of formula** | 19-0155.0091/F1 + 0,1% Tocopherol | 19-0155.0091/F1 + 1% Tocopherol |
| **Ingredient** | % w/w | |
| Vegetable Glycerine 4810 | 4 | 4 |
| Propylene Glycol | 20 | 20 |
| PEG-400 SR | 10 | 10 |
| Purified water | qs | qs |
| Brimonidine Tartrate | 0.15-3 | 0.15-3 |
| KOLLIDON VA 64 | 1 | 1 |
| XANTHAN GUM | 0,2 | 0,2 |
| POLAWAX NF | 10 | 10 |
| ARLAMOL PS11E-LQ-(RB) | 2.35 | 2.35 |
| DIMETHICONE (XIAMETER PMX-200) | 1 | 1 |
| KOLLICREAM OA | 2.5 | 2.5 |
| MARCOL 82 | 0.65 | 0.65 |
| BHA | | |
| DL TOCOPHEROL | 0,1 | 1 |
| IP PARABENES | 1 | 1 |
| NaOH 10% | 0.2 | 0.2 |
| Total | 100.00 | 100.00 |

### Example 3:

A topical brimonidine tartrate formulation (cf. example 1) was assessed for safety and effectiveness in a monocentric phase-1 study involving thirty-six (36) healthy volunteers.

The primary objective was to evaluate the local tolerance of four (4) increasing concentrations of brimonidine when applied once daily for twenty-one (21) days to the chest of healthy volunteers, in comparison with the reference product MIRVASO^{®} gel (0.5 % brimonidine tartrate), a vehicle and an untreated zone (UNT).

A secondary objective of this study was to measure the pharmacodynamic effect of these four (4) concentrations by the evaluation of their blanching effect on the skin, in comparison with the reference product MIRVASO^{®} gel (0.5% brimonidine tartrate), a vehicle and an untreated zone (UNT). This effect is directly linked to the mechanism of action of the drug which decreases the superficial blood flow by inducing a local vasoconstriction of the superficial dermal capillaries.

The skin blanching was measured via chromametry at day 1 (D1 - first application), and day 8 (D8) in a subgroup of participants. Results were expressed as the percent change in the chromametric a* value (measuring skin redness) when comparing the mean value (among six (6) healthy volunteers) at six (6) consecutive timepoints (at 2h, 4h, 6h, 8h, 10h and 12h) to the mean baseline value at T0h.

As illustrated by Figure 1, after the Day 1 application, the four (4) TAR-0520 hydrogel formulations of brimonidine tartrate appeared more performant than the marketed reference product MIRVASO^{®} gel (at same concentration of 0.5%).

However, surprisingly, after one (1) week of treatment, there is no change anymore in the a* value whatever the concentration and/or formulation considered (TAR-0520 formulations and MIRVASO^{®} gel) suggesting a tachyphylactic phenomenon.

Interestingly, this phenomenon was not reversed after only one day without product application. Importantly, this effect was seen with the hydrogel formulation and for the marketed comparator product containing the same active principle at same concentration, i.e. brimonidine tartrate 0.5%.

Thus, it is not a formulation effect and results from the active molecule brimonidine itself.

### Example 4:

A second study aimed at confirming this tachyphylactic phenomenon (dose effect and speed of appearance) and defining the time needed to full recovery of the drug effect (wash-out period needed to return to a full vasoconstriction response). This was a monocentric, investigator-blinded, pharmacology study exploring the pharmacodynamic effect of two concentrations of TAR-0520 gel (at 1.5% or 1%) applied at two different doses (2 or 4 mg/cm²) to two (2) small skin areas on the chest and two (2) small areas on the face in twenty-six (26) healthy volunteers.

In a first part of this study, TAR-520 gel was applied daily (except Saturdays and Sundays) for 3 consecutive weeks (fifteen (15) applications and two (2) wash-out periods of two (2) days).

In a second part, after a new treatment-free period of four (4) days (in a subgroup of thirteen (13) participants) or seven (7) days (in a subgroup of thirteen (13) participants) (as defined by a randomization list), a new single application of the same TAR-0520 gel dose and concentration was conducted on the same test areas.

This design thus allowed to confirm tachyphylaxis in a larger sample size, and to determine the minimal wash-out period necessary to reverse partially or completely the tachyphylaxic phenomenon.

The blanching effect was assessed clinically by the investigator and objectively using a chromameter (with a* value evaluating the skin redness).

This study confirmed the loss of blanching effect as early as the third day of treatment. While not clearly identified by the investigator (who still could see a change in skin color), it was significant on the a* value (Chromametry being known to be more sensitive than human eye).

This phenomenon was seen for the two (2) tested concentrations (1% and 1.5%), the two doses (2 and 4 mg/cm²) and the two (2) anatomical locations (Face and Chest).

As illustrated by Figure 2 (face area, concentration of 1.5% and 1% of brimonidine tartrate at a dose of 2 mg/cm²), a decrease in a* value is observed as early as D3.

At D8 and D15 (after 2 days of wash-out) the gain only reaches 30 to 50% of the initial response.

At day 23 (4 days of wash-out) and day 26 (7 days of wash-out), the response is not significantly different from baseline.

Thus, two (2) days of wash-out only result in a partial recovery when four (4) days or seven (7) days lead to a full recovery of a normal response.

As same as for the facial area, on chest area, as illustrated by Figure 3, two (2) days of wash-out result in a partial response and four (4) or seven (7) days lead to an almost full normal response.

It can be concluded that:
- a surprising loss of response amplitude is observed with continuous daily brimonidine topical applications;
- this loss is independent of concentration, dose, anatomical location or formulation;
- this loss does not recover after one (1) day of wash-out; and
- a partial response can be recovered after a wash-out period that is of at least two (2) days and a full response can be recovered after a wash-out period that is of at least three (3) days.

This finding is of high clinical relevance if the indication of topical brimonidine is the prevention of skin toxicities induced by systemic treatments such as chemotherapies. In that case a loss of vasoconstrictive potency would lead to an increased circulation in the capillaries with higher bioavailability of chemotherapy and an increased risk of skin toxicity.

## Claims

1. Composition comprising an alpha2-adrenoceptor agonist, for use as a medicament, an effective dose of said composition being topically administered to a patient in need thereof in series of daily applications of said composition, wherein a period of wash-out of at least two (2) days being applied between each series of applications during the all-treatment period.

2. Composition for use according to claim 1, wherein the period of wash-out is of more than two (2) days, preferably at least three (3) days, between each series of applications.

3. Composition for use according to claim 1 or 2, wherein the period of wash-out is comprised between three (3) days and seven (7) days.

4. Composition for use according to any of the preceding claims, wherein each series of daily applications have a duration of less than seven (7) days.

5. Composition for use according to claim 4, wherein each series of daily applications have a duration comprised between three (3) days and six (6) days, preferably of three (3), four (4) or five (5) days.

6. Composition for use according to any of the preceding claims, wherein each series of daily applications have a similar duration.

7. Composition for use according to any of the preceding claims, wherein the alpha2-adrenoceptor agonist is comprised at a concentration of between 0.15% and 3.00%, preferably between 0.75% and 2.50%, more preferably between 1.00% and 1.50%, by weight of the total weight of the composition.

8. Composition for use according to any of the preceding claims, wherein the alpha2-adrenoceptor agonist is brimonidine or its salts, preferably brimonidine tartrate.

9. Composition for use according to any of the preceding claims, in the prevention and/or treatment of skin damage induced by a systemic treatment, preferably a cancer treatment and more preferably a chemotherapy.

10. Composition for use according to any of the preceding claims, wherein the composition is in the form of a hydrogel, oil-in-water emulsion or water-in-oil emulsion, preferably hydrogel.

11. Composition for use according to claim 10, wherein the composition comprises a solvent phase comprising:
- polyethylene glycol in combination with propylene glycol and/or dimethyl sulfoxide;
- a hydrophilic film-forming agent chosen from a Polyvinylpyrrolidone/Vinyl Acetate copolymer, polyvinylpyrrolidone in a non-crosslinked or acetate form, taken alone or in combination;
- glycerin;
and in that it is in the form of a hydrogel.

12. Composition for use according to claim 11, wherein the composition comprises polyethylene glycol at a concentration of 10% by weight of the total weight of the composition in combination with propylene glycol at a concentration of 20% by weight of the total weight of the composition.

13. Composition for use according to claim 11 or 12, wherein the composition comprises a gelling agent chosen from xanthan gum and hydroxycellulose (HEC), taken alone or in combination.

14. Composition for use according to any of claims 11 to 13, wherein the composition comprises oleyl alcohol and vitamin E, taken alone or in combination.
